# EUROPÄISCHE PATENTSCHRIFT

(11) **EP 1 025 874 B1**
(45) Veröffentlichungstag und Bekanntmachung des Hinweises auf die Patenterteilung: **27.08.2003**
(21) Anmeldenummer: 99101965.4
(22) Anmeldetag: 01.02.1999
(51) Int. Cl.: A61M 16/04, A61F 2/20

(54) **Tracheostomaventil**
Tracheostomy valve
Valve de trachéotomie

(43) Veröffentlichungstag der Anmeldung: 09.08.2000
(73) Patentinhaber: Adeva Medical Gesellschaft für Entwicklung und Vertrieb von Medizinischen Implantat-Artikeln mbH, 23556 Lübeck (DE)
(72) Erfinder: Geertsema, Albert, 9714 Gn Groningen (NL)
(74) Vertreter: Fuchs Mehler Weiss & Fritzsche

(56) Entgegenhaltungen:
- EP-B- 0 221 973
- EP-B- 0 617 630
- DE-A- 2 253 496
- GB-A- 2 164 424
- US-A- 3 827 440

## Beschreibung

Die vorliegende Erfindung betrifft ein Tracheostomaventil. Derartige Ventile sind bekannt beispielsweise aus der EP-B-0 221 973 oder aus der EP-B-0 617 630.

Bei dem in der letztgenannten Druckschrift genannten Tracheostomaventil handelt es sich um ein solches mit einem Ventilschließglied, das unter Ansprechen auf eine Luftverschiebung das Ventil schließt. Dies wird vom Patienten veranlaßt, um die Luft durch ein Shuntventil zwischen Trachea und Oesophagus leiten zu können, um so eine gewisse Sprechmöglichkeit aufrechtzuerhalten. Das erwähnte Ventil weist darüber hinaus ein sogenanntes Abblasventilglied auf, das sich unter Ansprechen auf einen besonderen Überdruck auf der Tracheaseite des Ventils öffnet. Beim Husten ist somit dafür Sorge getragen, daß sich das Abblasventilglied öffnet und die Luft und Sputum aus dem Stoma herausgepreßt werden kann. Diese Funktion ist wichtig, da es ansonsten zu schweren Komplikationen kommen kann, wenn beispielsweise das Sekret (Sputum) in die Lungenflügel gelangt, wo es zu schweren Infektionen führen kann.

Bei dem bekannten Ventil weist das Abblasventilglied eine Öffnung auf, die durch das erstgenannte Ventilschließglied schließbar ist.

Hierdurch erst kann die Luft so umgeleitet werden, daß sie durch das genannte Shuntventil geführt werden kann.

Wenn der Patient sprechen möchte, muß also ein Teil des Luftstromes beim Exhalieren für die Ansteuerung des genannten Ventilschließgliedes dienen, so daß dieses in seine Schließstellung gebracht werden kann. Darüber hinaus muß der Patient das Ventilschließglied im Sprechbetrieb jedesmal pro Atemzug ansteuern, damit er nach jeder Inhalation das Ventilschließglied wieder in die Schließstellung bringen kann, um den Luftstrom durch das Shuntventil leiten zu können.

Wenn das bekannte Ventil auch erhebliche Vorteile gegenüber älteren Ventilkonstruktionen aufweist, wird das jedesmalige Ansteuern des Ventilschließgliedes im Sprechbetrieb von dem Patienten als unkomfortabel empfunden.

Die Aufgabe der vorliegenden Erfindung besteht daher darin, den Komfort des Patienten bei gleichbleibender Sicherheit zu erhöhen.

Gelöst wird diese Aufgabe durch ein Tracheostomaventil mit den Merkmalen des Anspruchs 1. Vorteilhafte Weiterbildungen und Ausführungsformen ergeben sich aus den Unteransprüchen.

Demgemäß ist vorgesehen, daß das Tracheostomaventil ein Ventilgehäuse aufweist, in dem eine Ventilklappe schwenkbar gelagert ist. Diese ist auf eine Luftströmung vorbestimmbarer Stärke bei Inhalationen in eine das freie Lumen des Ventilgehäuses abdichtende Stellung und auf eine Luftströmung vorbestimmbarer Stärke bei Exhalation in eine das freie Lumen wenigstens teilweise wieder freigebende Stellung verschwenkbar. Es weist darüber hinaus ein in der Ventilklappe integriertes Bypassventilglied auf, durch welches bei in Schließstellung befindlicher Ventilklappe Atemluft inhaliert werden kann, wobei die beiden ersten Schaltpunkte der Ventilklappe zwischen der abdichtenden und der das freie Lumen des Ventilgehäuses wenigstens teilweise freigebenden Stellung voreinstellbar sind.

Das erfindungsgemäße Ventil schließt also mit seiner Ventilklappe bei einem verstärktem Einatmungsvorgang, wohingegen das Ventil gemäß der erwähnten EP-B-0 617 630 bei einem verstärktem Ausatmen schließt. Hingegen muß das erfindungsgemäße Ventil durch einen verstärkten Ausatemdruck wieder geöffnet werden, was aber dem Sicherheitsaspekt zuträglich ist, wenn beim Husten etc. ein erhöhter Druck in der Trachea aufgebaut wird, der über das Ventil abgebaut werden muß. Hierbei übernimmt die Ventilklappe des erfindungsgemäßen Tracheostomaventils insoweit auch die Funktion des Abblasventilgliedes beim Ventil gemäß der vorerwähnten Druckschrift.

Durch ein verstärktes Inhalieren schaltet der Patient das Tracheostomaventil in den Sprechbetrieb. Bei der nächstmaligen Exhalation entweicht keine Luft aus dem Ventil durch das Stoma, sondern wird durch den Shunt bzw. durch das entsprechende Shuntventil geleitet, wonach sie zur Lautbildung und Artikulation zur Verfügung steht. Möchte der Patient das Ventil weiterhin im Sprechbetrieb halten, kann er über das Bypassventilglied erneut inhalieren, ohne daß die Ventilklappe selbst kurzfristig und kurzzeitig in die das freie Lumen des Ventilgehäuses zumindest teilweise freigebende Stellung verschwenkt werden müßte. Nach erfolgter Inhalation wird Luft sodann wieder am Stoma vorbei durch das Shuntventil geleitet.

Es muß vorliegend also nicht bei jedem Atmenzug, wie beim Ventil gemäß dem Stand der Technik, das dort genannte Ventilschließglied angesteuert werden, um weitere Atemzüge zu tätigen. Hierfür sorgt das Bypassventilglied, welches zum Schalten - nämlich zum Schließen beim Ansetzen zum Sprechen - nur die Bewegung einer relativ kleinen und leichten Ventilscheibe benötigt.

Gemäß einer besonders bevorzugten Ausführungsform ist vorgesehen, daß an der Ventilklappe mindestens zwei Permanentmagneten angeordnet sind, derart, daß der eine Permanentmagnet mit einem ersten im Ventilgehäuse angeordneten metallischen Schieber einen Magnetverschluß in der abdichtenden Stellung der Ventilklappe und der zweite Permanentmagnet mit einem zweiten im Ventilgehäuse angeordneten Schieber einen Magnetverschluß in der das freie Lumen des Ventilgehäuses wenigstens teilweise freigebenden Stellung der Ventilklappe bildet. Diese Ausführungsform bietet eine sehr komfortable Möglichkeit, die schon erwähnten Schaltpunkte der Ventilklappe einzustellen, dadurch, daß die Haltekraft der Magnetverschlüsse durch Betätigung des jeweiligen Schiebers erhöht oder erniedrigt werden kann, indem der Kontakt zwischen Permanentmagnet und metallischem Schieber abhängig von der Stellung des Schiebers größer oder kleiner ist und somit die entsprechende Haltekraft, wie an sich aus dem Stand der Technik schon bekannt.

Eine besonders bevorzugte Weiterbildung sieht vor, daß das Bypassventilglied als Rückschlagventil ausgebildet ist. Das Rückschlagventil muß beim Inhalationsvorgang selbständig öffnen und beim Exhalationsvorgang entsprechend schließen. Das Rückschlagventil kann dabei selbstschließend ausgebildet sein, so daß der Patient also praktisch überhaupt keine Kraft bzw. keinen Teilluftstrom aufwenden muß, um das Bypassventilglied beim Exhalationsvorgang zu schließen, um das gesamte Tracheostomaventil im Sprechbetrieb zu halten.

Besonders bevorzugt wird eine Ausführungsform, bei der die Ventilklappe im Ventilgehäuse exzentrisch aufgehängt ist, worunter verstanden wird, daß der Schwenkpunkt der Ventilklappe unterhalb der Längsachse des Ventilgehäuses liegt. Damit kann erreicht werden, daß die beim jeweiligen Inhalations- oder Exhalationsvorgang dem jeweiligen Luftstrom effektiv ausgesetzte Fläche der Ventilklappe am wirksamsten angesteuert werden kann, um das für die Schwenkbewegung benötigte Drehmoment (um den Schwenkpunkt) erzeugen zu können.

An der Ventilklappe kann ein Anschlag zur Begrenzung der Schwenkbewegung angeformt sein, um die Ventilklappe stets in einer definierten Lage zu halten.

Weist die Ventilklappe einen solchen Anschlag aus, so ist besonders bevorzugt vorgesehen, daß der schon erwähnte zweite Permanentmagnet bei der Ausführung mit den Magnetverschlüssen, der für einen Magnetverschluß in der das freie Lumen des Ventilgehäuses wenigstens teilweise freigebenden Stellung der Ventilklappe vorgesehen ist, am Ende des Anschlags angeordnet ist. Der erste Permanentmagnet, welcher mit der anderen Stellung der Ventilklappe korrespondiert, kann dann an einer Stirnkante der Ventilklappe eingelassen sein.

Die Erfindung wird nachfolgend anhand eines Ausführungsbeispiels gemäß der Zeichnungsfiguren näher erläutert. Hierbei zeigt:
- Figur 1: eine Schnittansicht des Tracheostomaventils mit der Ventilklappe in der das freie Lumen des Ventils abdichtenden Stellung,
- Figur 2: eine Ansicht des Ventils in Richtung des Pfeiles II in Figur 1,
- Figur 3: das Ventil mit der Ventilklappe in einer Stellung, bei der das Lumen teilweise freigegeben ist, und
- Figur 4: eine Ansicht des Ventils in Richtung des Pfeiles IV in Figur 3.

Nachfolgend sind dieselben Bezugszeichen für die jeweils gleichen Teile verwendet.

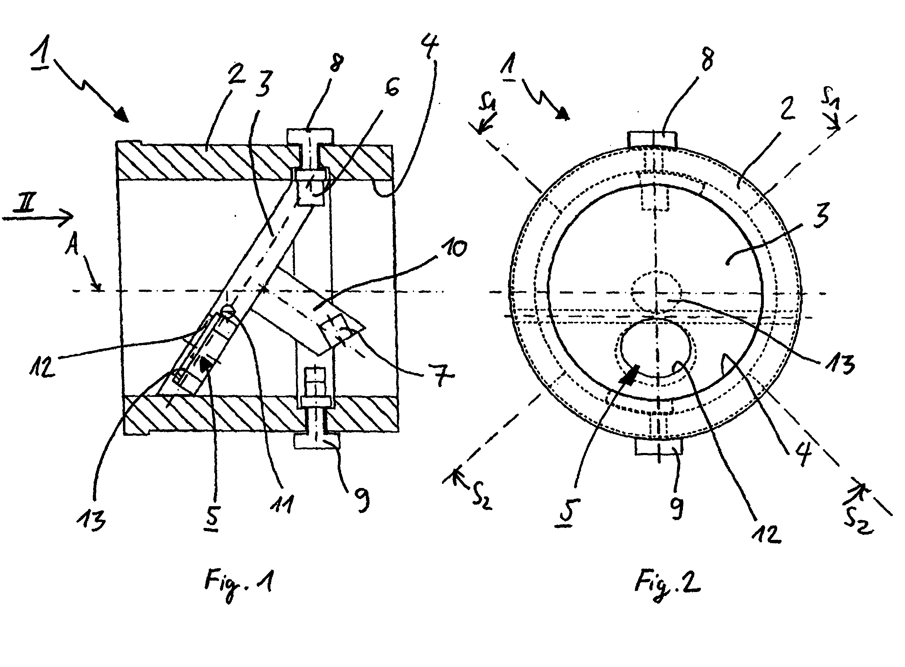

Fig. 1 zeigt nun die Schnittansicht des Tracheostomaventils 1. Das Tracheostomaventil weist ein Ventilgehäuse 2 auf, welches vorliegend als zylindrisches Rohr dargestellt ist. Das Ventilgehäuse 2 weist ein freies Lumen 4 auf, durch welches Luft von der Außenseite (rechts in Fig. 1) in ein tracheaseitiges Kanülenstück (links in Fig. 1) strömen kann.

Im Inneren des Ventilgehäuses 2 ist eine Ventilklappe 3 gelagert, und zwar um einen exzentrisch gelagerten Schwenkpunkt 11. Dieser liegt also unterhalb der Längsachse A des Ventilgehäuses 2.

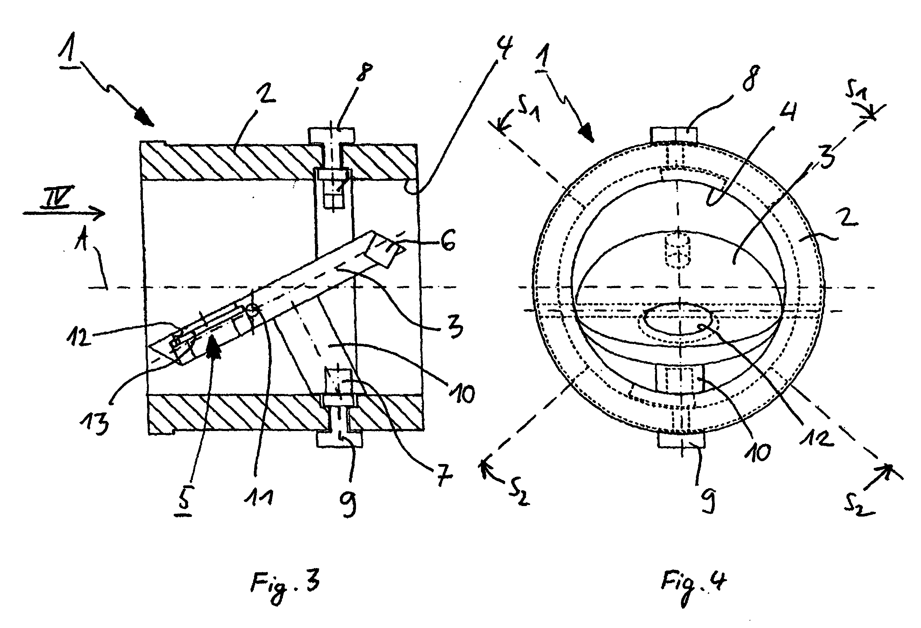

An die Ventilklappe 3 ist ein Anschlag 10 angeformt, der eine Schwenkbewegung von der Lage gemäß Fig. 1 in eine Lage gemäß Fig. 3 begrenzt.

Im Ventilgehäuse 2 ist ein erster metallischer Schieber 8 angeordnet, der in einem Schwenkbereich S₁ (Fig. 2) auf dem Ventilgehäuse 2 verfahren werden kann. Der metallische Schieber 8 bildet zusammen mit einem Permanentmagneten 6, der am Ende der Ventilklappe 3 angeordnet ist, einen Magnetverschluß, dessen Haltekraft von der Lage des Magnetschiebers 8 abhängt.

Im Ventilgehäuse 2 ist darüber hinaus ein entsprechend ausgebildeter zweiter metallischer Schieber 9 angeordnet, der in einem Schwenkbereich S₂ (Fig. 2) entsprechend verfahrbar ist. Der zweite Schieber 9 bildet zusammen mit einem Permanentmagneten 7, der am Ende des Anschlags 10 angeordnet ist, einen zweiten Magnetverschluß, dessen Haltekraft entsprechend abhängt von der Lage des Schiebers 9 in bezug auf den Permanentmagneten 7.

Abhängig von der Schaltposition der Ventilklappe 3 ist der eine oder andere Magnetverschluß aktiv. In der in Fig. 1 dargestellten Lage, in der die Ventilklappe 3 das freie Lumen 4 des Ventils 1 abdichtet, ist der Magnetverschluß aus dem ersten Permanentmagneten 6 am Ende der Ventilklappe 3 sowie aus dem ersten Schieber 8 aktiv, d.h., er hält die Ventilklappe 3 so lange in der dargestellten Stellung, bis eine die Haltekraft übersteigende Kraft an der Ventilklappe 3 aufgrund einer Luftströmung angreift.

Dies ist ausgehend von der Stellung der Ventilklappe 3 in Fig. 1 dann der Fall, wenn durch ein verstärktes Exhalieren eine die Haltekraft des ersten Magnetverschluß 6, 8 übersteigende Kraft wirkt, um die Ventilklappe 3 in die Lage gemäß der Fig. 3 zu verschwenken. Hierzu strömt in Fig. 1 ein starker Luftstrom von links nach rechts, so daß die Ventilklappe 3 die Stellung in Fig. 3 annimmt. Während Fig. 1, die den Sprechbetrieb des Tracheostomaventils 1 darstellt, ist in Fig. 3 der normale Atembetrieb dargestellt.

In der Ventilklappe 3 ist integriert ein Bypassventil 5, welches vorliegend aus einem Rückschlagventil mit einer Öffnung 12 und einer Ventilklappe 13 gebildet wird.

Ist das Tracheostomaventil 1 im normalen Atembetrieb, übt das Bypassventil 5 praktisch keine Funktion aus. Luft wird vom Patienten durch das von der Ventilklappe 3 freigegebene Teillumen des gesamten Lumens 4 des Ventilgehäuses 2 inhaliert und exhaliert (Fig. 4).

Möchte der Patient nun das Ventil in den Sprechbetrieb, wie in Fig. 1 dargestellt, schalten, so inhaliert er etwas stärker als im normalen Atembetrieb, so daß dann die Haltekraft des Magnetschlusses 7, 9 nicht mehr ausreicht, die Ventilklappe 3 in der in Fig. 3 dargestellten Lage zu halten. Die Ventilklappe 3 schwenkt vielmehr in die Lage gemäß Fig. 1, wo sie durch den Magnetverschluß 6, 8 gehalten wird. Die normale Atemfunktion wird in dieser Lage der Ventilklappe 3 durch das Bypassventil 5 ausgeübt, wo die kleine Ventilklappe 13 beim Inhalieren die Ventilöffnung 12 freigibt. Das Bypassventil 5 nimmt seine Funktion also im wesentlichen im Sprechbetrieb des Tracheostomaventils 1 wahr. Ist das Tracheostomaventil 1 einmal in Sprechbetrieb (Fig. 1) geschaltet, braucht der Patient praktisch keinen Teilluftstrom mehr pro Atemzug, um das Tracheostomaventil 1 in den Sprechbetrieb, in dem es sich jetzt bereits befindet, zu bringen, wodurch der Komfort des Patienten beim Sprechen erheblich erhöht wird.

Möchte der Patient nach dem Sprechen das Ventil 1 nun wieder in den normalen Atembetrieb (Fig. 3) überführen, so exhaliert er etwas stärker als im normalen Atembetrieb, wodurch die Haltekraft des Magnetverschlusses 6, 8 nicht mehr ausreicht, um die Ventilklappe 3 in der Stellung gemäß Fig. 1 zu halten. Diese schwenkt vielmehr wieder zurück in die Stellung gemäß Fig. 3.

## Patentansprüche

1. Tracheostomaventil mit einem Ventilgehäuse (2), in dem eine Ventilklappe (3) schwenkbar gelagert ist, die auf eine Luftströmung vorbestimmbarer Stärke bei Inhalation in eine das freie Lumen (4) des Ventilgehäuses (2) abdichtende Stellung und auf eine Luftströmung vorbestimmbarer Stärke bei Exhalation in eine das freie Lumen (4) wenigstens teilweise wieder freigebende Stellung verschwenkbar ist, und mit einem in der Ventilklappe (3) integriertem Bypassventilglied (5), durch welches bei in Schließstellung befindlicher Ventilklappe (3) Atemluft inhaliert werden kann, wobei die beiden Schaltpunkte der Ventilklappe (3) zwischen der abdichtenden und der das freie Lumen (4) des Ventilgehäuses (2) wenigstens teilweise freigebenden Stellung voreinstellbar sind.

2. Tracheostomaventil nach Anspruch 1,
bei dem an der Ventilklappe (3) wenigstens zwei Permanentmagneten (6, 7) angeordnet sind, derart, daß der eine Permanentmagnet (6) mit einem ersten im Ventilgehäuse (2) angeordneten metallischen Schieber (8) einen Magnetverschluß in der abdichtenden Stellung der Ventilklappe (3) und der zweite Permanentmagnet (7) mit einem zweiten im Ventilgehäuse (2) angeordneten Schieber (9) einen Magnetverschluß in der das freie Lumen (4) des Ventilgehäuses (2) wenigstens teilweise freigebenden Stellung der Ventilklappe (3) bildet.

3. Tracheostomaventil nach Anspruch 1 oder 2, bei dem das Bypassventilglied (5) als Rückschlagventil (12, 13) ausgebildet ist.

4. Tracheostomaventil nach einem der Ansprüche 1 bis 3, bei dem die Ventilklappe (3) im Ventilgehäuse (2) exzentrisch aufgehängt ist, derart, daß der Schwenkpunkt (11) der Ventilklappe (3) unterhalb der Längsachse (A) des Ventilgehäuses (2) liegt.

5. Tracheostomaventil nach einem der Ansprüche 1 bis 4, bei dem an der Ventilklappe (3) ein Anschlag (10) zur Begrenzung der Schwenkbewegung angeformt ist.

6. Tracheostomaventil nach den Ansprüchen 2 und 5, bei dem am Ende des Anschlags (10) der zweite Permanentmagnet (7) angeordnet ist.

## Claims

1. Tracheostoma valve having a valve housing (2), in which a valve flap (3) is pivotably mounted, which can be pivoted into a position sealing the free lumen (4) of the valve housing (2) with an air stream of pre-determinable strength during inhalation and into a position at least partly re-releasing the free lumen (4) with an air stream of pre-determinable strength during exhalation, and having a bypass valve member (5) integrated in the valve flap (3), through which bypass valve member (5) respiratory air may be inhaled when the valve flap (3) is situated in the closed position, wherein the two switching points of the valve flap (3) between the sealing position and the position at least partly releasing the free lumen (4) of the valve housing (2) can be preset.

2. Tracheostoma valve according to claim 1, in which at least two permanent magnets (6, 7) are arranged on the valve flap (3), such that the one permanent magnet (6) forms a magnetic closure in the sealing position of the valve flap (3) with a first metallic slide valve (8) arranged in the valve housing (2) and the second permanent magnet (7) forms a magnetic closure in the position of the valve flap (3) at least partly releasing the free lumen (4) of the valve housing (2) with a second slide valve (9) arranged in the valve housing (2).

3. Tracheostoma valve according to claim 1 or 2, in which the bypass valve member (5) is designed as a nonreturn valve (12, 13).

4. Tracheostoma valve according to one of claims 1 to 3, in which the valve flap (3) is suspended eccentrically in the valve housing (2), such that the pivoting point (11) of the valve flap (3) lies below the longitudinal axis (A) of the valve housing (2).

5. Tracheostoma valve according to one of claims 1 to 4, in which a stop (10) for limiting the pivoting movement is moulded onto the valve flap (3).

6. Tracheostoma valve according to claims 2 and 5, in which the second permanent magnet (7) is arranged on the end of the stop (10).

## Revendications

1. Valve de trachéostomie comprenant une cage de valve (2) dans laquelle est logé de manière pivotante un clapet (3), qui peut pivoter, à la suite d'un flux d'air d'intensité prédéfinissable pendant une inhalation, dans une position fermant de manière étanche la lumière (4) libre de la cage de valve (2) et, à la suite d'un flux d'air d'intensité prédéfinissable pendant une exhalaison, vers une position ouvrant à nouveau au moins partiellement la lumière (4) libre, et comprenant un élément de dérivation (5), qui est intégré dans le clapet de valve (3) et qui permet d'inhaler l'air lorsque le clapet de valve (3) est en position de fermeture, dans laquelle valve il est possible de prérégler les deux points de manoeuvre du clapet de valve (3) entre la position fermant de manière étanche et celle ouvrant au moins partiellement la lumière (4) libre.

2. Valve de trachéostomie selon la revendication 1, dans laquelle au moins deux aimants permanents (6, 7) sont disposés au niveau du clapet de valve (3), de telle sorte que l'un des aimants permanents (6) forme avec une première coulisse (8) métallique, agencée dans la cage de valve (2), une fermeture magnétique dans la position de fermeture étanche du clapet de valve (3) et le deuxième aimant permanent (7) forme avec une deuxième coulisse (9), agencée dans la cage de valve (2), une fermeture magnétique dans la position du clapet de valve (3) ouvrant au moins partiellement la lumière (4) libre de la cage de valve (2).

3. Valve de trachéostomie selon la revendication 1 ou 2, dans laquelle l'élément de dérivation (5) est conçu sous forme de clapet de non-retour (12, 13).

4. Valve de trachéostomie selon l'une quelconque des revendications 1 à 3, dans laquelle le clapet de valve (3) est accroché de manière excentrée dans la cage de valve (2), de telle sorte que le point de pivotement (11) du clapet de valve (3) est situé en dessous de l'axe longitudinal (A) de la cage de valve (2).

5. Valve de trachéostomie selon l'une quelconque des revendications 1 à 4, dans laquelle une butée (10), destinée à limiter le mouvement de pivotement, est formée au niveau du clapet de valve (3).

6. Valve de trachéostomie selon les revendications 2 et 5, dans laquelle le deuxième aimant permanent (7) est agencé au niveau de l'extrémité de la butée (10).
